# EUROPEAN PATENT APPLICATION

(11) **EP 2 153 839 A1**
(43) Date of publication of application: **17.02.2010**
(21) Application number: 08752119.1
(22) Date of filing: 25.04.2008
(51) Int. Cl.: A61K 38/00, A61K 31/7004, A61K 31/7016, A61P 1/04, A23C 9/13, A23G 1/00, A23G 1/30, A23L 1/30, A23L 2/38

(54) **COMPOSITION FOR PREVENTION AND/OR TREATMENT OF PEPTIC ULCER**

(30) Priority: 27.04.2007 JP 2007119782
(71) Applicant: Nisshin Pharma Inc., Tokyo 101-8441 (JP)
(72) Inventor: NAGASE, Masao, Fujimino-shi Saitama 356-8511 (JP); HIRAMOTO, Shigeru, Fujimino-shi Saitama 356-8511 (JP); HARATA, Masataka, Fujimino-shi Saitama 356-8511 (JP); SUZUKI, Yoshio, Tokyo 101-8441 (JP)
(74) Representative: Schrell, Andreas
(86) International application number: PCT/JP2008/058045
(87) International publication number: WO 2008/136397

(57) **Abstract**

A composition for effectively preventing or treating peptic ulcers not caused by *Helicobacter pylori* that does not cause any adverse reaction and thus is highly safe is provided. The present invention relates to a composition for preventing and/or treating peptic ulcers not caused by *Helicobacter pylori,* which comprises as an active ingredient a browning reaction product of a sugar and a protein.

## Description

### Technical Field

The present invention relates to a composition for preventing and/or treating peptic ulcers. More specifically, the present invention relates to a composition for preventing and/or treating peptic ulcers that are not caused by *Helicobacter pylori,* such as gastric ulcers or duodenal ulcers that are caused by NSAIDs or stress.

### Background Art

Among gastrointestinal disorders, peptic ulcers are the most prevalent, and have been found to affect several percent (%) of participants in general health checkups. Peptic ulcers are disorders that both doctors and patients tend to encounter. Regarding causes of gastric ulcers, it has been revealed in recent years that most peptic ulcer cases are due to *Helicobacter pylori* (hereinafter, also referred to as *H. pylori*) as a result of discoveries and studies concerning *H. pylori.* However, not a few patients often develop ulcers independently of *H. pylori.* That is, many patients who are not infected with *H. pylori* often develop ulcers. These patients' ulcers cannot be cured even by *H. pylori* eradication therapy, so that this poses a problem as the *H. pylori* eradication therapy spreads.

NSAIDs (that is, non-steroidal anti-inflammatory drugs) are broadly used worldwide for anti-inflammation and analgesic effects. NSAID strongly suppress inflammation reactions, but result in high incidence of peptic ulcers as complications. The incidence of peptic ulcers in the duodenum is said to be several percent (%), but the same in the stomach is said to be more than a dozen to several tens of percent (%). NSAID ulcers are also said to account for 20% to 40% of peptic ulcer cases. NSAIDs have analgesic effects, so that NSAID ulcers often advance unnoticed, which can lead to the sudden discovery of gastrointestinal bleeding, gastric perforation, or the like.

Aspirin is the most famous example of an NSAID. It is broadly used as a cyclooxygenase inhibitor, a platelet aggregation inhibitor, an analgesic, or the like. Upper gastrointestinal bleeding due to aspirin is confirmed very often. It has been reported that gastric mucosal damage due to aspirin is dose-related.

Meanwhile, there are many ulcer cases due to neither *H. pylori* nor NSAIDs. Such ulcers are indicated to be caused by hereditary factors, acid hyper-secretion, lowered defensive functions, delayed ability of gastric emptying, stress, smoking, Zollimger-Ellison syndrome, and the like. Ulcers due to such causes are resistant to various therapeutic methods and thus require long-term management. Peptic ulcers due to stress, such as gastric ulcers and duodenal ulcers due to stress, form a group of diseases including acute gastric mucosal lesions, hemorrhagic gastritis, acute erosive gastritis, and acute gastric ulcers, in which lesions advance rapidly. Such peptic ulcers due to stress occur frequently in males. It is thought that in persons under high social stress or persons who are personally nervous, gastric juice secretion or gastric blood flow is affected by stress via autonomic nerves, so that ulcers are formed. Hence, people today under a great deal of stress may be repeatedly affected by stress ulcers, and this poses a significant problem.

Furthermore, it has been reported that reflux esophagitis known as an esophageal ulcer or gastroesophageal reflux disease involving no finding of inflammation but showing subjective symptoms such as chest pain is caused by increased gastric-acid secretion, hypotonia of gastroesophageal sphincter, or the like. It is also said that gastric-acid secretion is recovered when *H. pylori* is eradicated, causing reflux esophagitis, gastroesophageal reflux disease, or the like.

As described above, even after the discovery of *Helicobacter pylori* as a causative microorganism of peptic ulcers, there are many peptic ulcer cases that are not caused by *H. pylori* or peptic ulcer cases that occur after *H*. *pylori* eradication. Sufficient preventive and therapeutic methods are not provided for such patients under current circumstances. Accordingly, as *H*. *pylori* eradication technology advances, peptic ulcers not caused by *Helicobacter pylori* are emerging at the forefront.

Various therapeutic agents have been used for treatment of peptic ulcers. However, neither NSAID ulcers nor stress ulcers can be sufficiently cured by the current mainstream therapy, *H. pylori* eradication. Also, the use of other medicaments could cause drowsiness or appearance of an adverse reaction such as anaphylactic shock. Moreover, long-term ingestion of pharmaceutical products is problematic in terms of safety and healthcare, and requires repeated hospital visits, which is associated with great difficulties.

The present inventors have studied a substance that inhibits adhesion of *H. pylori* to gastric mucosa for the purpose of preventing and treating peptic ulcers due to *H. pylori,* which is a social problem. The present inventors have discovered that a browning reaction product of a sugar and a protein has the above effects and have applied for a patent (International Patent Publication WO03/063886 Pamphlet). However, the importance of the prevention and treatment of peptic ulcers not caused by infection with *H. pylori* is growing, as described above. Thus, development of food-ingredient-derived prophylactics and therapeutic agents for peptic ulcers, which can be conveniently ingested safely without causing any adverse reaction, has been required.

### Disclosure of the Invention

An object of the present invention is to provide a composition for effectively preventing and/or treating peptic ulcers not caused by *Helicobacter pylori* that does not cause any adverse reaction and thus is highly safe.

The present inventors have discovered that a browning reaction product of a sugar and a protein has an effect of inhibiting adhesion of *Helicobacter pylori* to gastric mucosa. Through further examinations, the present inventors have discovered that a browning reaction product of a sugar and a protein exerts extremely high preventive and therapeutic effects against peptic ulcers not caused by *Helicobacter pylori,* and thus they have completed the present invention.

The present invention is as follows.
(1) A composition for preventing and/or treating a peptic ulcer not caused by *Helicobacter pylori,* comprising as an active ingredient a browning reaction product of a sugar and a protein.
(2) The composition according to (1), wherein the peptic ulcer is an NSAID ulcer or a stress ulcer.
(3) The composition according to (1) or (2), wherein the weight-average molecular weight of the browning reaction product of a sugar and a protein ranges from 4,000,000 to 12,000,000.

According to the present invention, a composition effective for prevention and/or treatment of peptic ulcers not caused by *Helicobacter pylori* and particularly NSAID ulcers and stress ulcers, is provided. The composition of the present invention can be continuously ingested daily, since it causes no adverse reaction, has high safety, and is a food-derived ingredient.

This description includes part or all of the contents as disclosed in the description and/or drawings of Japanese Patent Application No. 2007-119782, which is a priority document of the present application.

### Best Mode for Carrying Out the Invention

The composition of the present invention for preventing and/or treating peptic ulcers not caused by *Helicobacter pylori* will be described in detail below.

In the present invention, the term "a peptic ulcer(s) not caused by *Helicobacter pylori*" refers to an ulcer in the gastrointestinal tract, which is not caused by *Helicobacter pylori.* Examples of such ulcer include ulcers in the gastrointestinal tract where no *Helicobacter pylori* exists, ulcers in the gastrointestinal tract that has been subjected to *Helicobacter pylori* eradication, and peptic ulcers not caused by *Helicobacter pylori,* but developed in association with peptic ulcers caused by *Helicobacter pylori.*

Examples of peptic ulcers include esophageal ulcers, gastric ulcers, duodenal ulcers, and small intestine ulcers. The term "peptic ulcer" generally refers to a condition wherein partial defects of the epithelial tissue of gastrointestinal mucosa reach the submucosa. In the present invention, examples of peptic ulcers also include pre-ulcer stage symptoms, such as gastrointestinal inflammation and symptoms due to mucosal damage of gastrointestinal tracts (e.g., esophagus, stomach, duodenum, and small intestine), such as breast discomfort, abdominal discomfort, heavy stomach feeling, and abdominal distension. Examples of peptic ulcers not caused by *Helicobacter pylori* include NSAID ulcers, ulcers caused by neither *H. pylori* nor NSAIDs, and peptic ulcers, reflux esophagitis, and gastroesophageal reflux disease occurring after *H. pylori* eradication therapy.

The term "NSAID ulcer" refers to a peptic ulcer that is caused by administration of a non-steroidal anti-inflammatory drug (NSAID) and particularly a gastric ulcer and a duodenal ulcer. Examples of NSAIDs include aspirin, diclofenac, diflusinal, etodolac, fenbufen, fenoprofen, flufenisal, flurbiprofen, ibuprofen, indomethacin, ketoprofen, ketorolac, meclofenamic acid, mefenamic acid, meloxicam, nabumetone, naproxen, nimesulide, nitroflurbiprofen, olsalazine, oxaprozin, phenyl butazone, piroxicam, sulfasalazine, sulindac, tolmetin, and zomepirac. Therefore, examples of NSAID ulcers also include peptic ulcers that are caused by these examples, such as gastrointestinal bleeding and gastrointestinal mucosal damage.

Examples of ulcers caused by neither *H. pylori* nor NSAIDs include hereditary ulcers, ulcers due to acid hypersecretion, ulcers due to lowered defensive functions, ulcers due to delayed ability of gastric emptying, ulcers due to stress (stress ulcers), ulcers due to smoking, and Zollimger-Ellison syndrome. The term "stress ulcer" refers to a peptic ulcer, particularly refers to a gastric ulcer and a duodenal ulcer, that is formed when gastric juice secretion or gastric blood flow is affected by stress via autonomic nerve. Examples of stress ulcers include acute gastric mucosal lesions, hemorrhagic gastritis, acute erosive gastritis, and acute gastric ulcers.

A browning reaction product of a sugar and a protein, which is an active ingredient in the composition of the present invention is a product of browning reaction (also referred to as amino-carbonyl reaction or Maillard reaction) between a sugar and a protein. Specifically, such browning reaction product is a polymeric substance (sugar-protein polymerized product) comprising macromolecular melanoidin as a major ingredient. Maillard reaction between amino groups of a protein and a reducing sugar is conducted to generate unstable Schiff base. The reaction further proceeds to generate very many types of compound such as α-ketoaldehyde, diketones, furfural, furanone, and pyrrole aldehyde. These compounds react again with amino groups of the protein, and polymerization is repeated to form melanoidin. Melanoidin is a generic name for various types of pigmentary polymerized product. As the browning reaction proceeds to increase the amount of melanoidin generated, ultraviolet absorption and visible light absorption are increased to further browning. A browning reaction can be carried out by suspending a mixture that contains various types of sugar and various types of protein, such as a sugar and a protein, in an aqueous solution, so as to prepare an aqueous suspension (containing an aqueous solution), and then heating the suspension. For example, a browning reaction can be carried out according to the production procedures for a browning reaction product as described in the International Patent Publication WO03/063886 Pamphlet.

A browning reaction can be carried out in a neutral aqueous solution or an aqueous alkaline solution. However, since the browning reaction is accelerated in an aqueous alkaline solution, such browning reaction is preferably carried out in an aqueous alkaline solution. As aqueous alkaline solutions, various aqueous alkaline solutions can be used, such as an aqueous sodium hydroxide solution, an aqueous potassium hydroxide solution, an aqueous sodium hydrogencarbonate (*juso*) solution, an aqueous sodium carbonate solution, and an aqueous disodium hydrogenphosphate solution. The concentration of such aqueous alkaline solution is not particularly limited. For example, a 0.01-0.5 N aqueous alkaline solution can be used. Any amount of an aqueous alkaline solution may be employed herein, as long as a mixture of a sugar and a protein or a material containing a sugar and a protein can be sufficiently suspended homogenously therein. For example, such mixture or material may be suspended in 1-100 times its volume and preferably 5-20 times its volume, of an aqueous alkaline solution.

The browning reaction is carried out until absorbance at 405 nm measured for an aqueous suspension (containing an aqueous solution) containing 5% by mass of a sugar and a protein together reaches 0.01 or more and preferably 0.1 or more. The fact that absorbance at 405 nm is 0.01 or more indicates sufficient progress of the browning reaction. Absorbance is measured by measuring 100 µl of an aqueous suspension using a microplate reader, for example. The term "absorbance at 405 nm" when measurement is carried out for an aqueous suspension (containing an aqueous solution) containing 5% by mass of a sugar and a protein together refers to absorbance obtained via conversion in terms of an aqueous suspension containing 5% by mass of a sugar and a protein together. For example, when the browning reaction of an aqueous suspension containing 10% by mass of a sugar and a protein together is carried out, the reaction is continued until absorbance at 405 nm (measured after the reaction solution has been diluted twofold) reaches the above level. Here, examples of an aqueous suspension containing a sugar and a protein also include a material containing a sugar and a protein, such as a food product containing a sugar and a protein and an aqueous suspension obtained by suspension of such material in an aqueous solution. Therefore, when a browning reaction is carried out for a food product containing a sugar and a protein or an aqueous suspension thereof, the total mass of the sugar and the protein is calculated similarly and then absorbance is measured after the total mass is converted in terms of an aqueous suspension containing 5% by mass of the sugar and the protein together.

Regarding conditions for a browning reaction, when the browning reaction is carried out in a neutral aqueous solution, the reaction temperature is 100°C or higher and is particularly preferably 120°C or higher and the reaction time is generally 20 minutes or more and preferably ranges from 30 minutes to 10 hours. Also, when a browning reaction is carried out in an aqueous alkaline solution, the reaction temperature ranges from room temperature to 100°C and particularly preferably ranges from 40°C to 100°C and the reaction time generally ranges from 30 minutes to 20 hours and preferably ranges from 2 to 8 hours.

A browning reaction product is a polymer that is obtained by successive polymerization of a sugar and a protein as described above, so that it has a wide-ranging molecular weight distribution. A material that is preferably used in the present invention contains a browning reaction product exhibiting a high degree of polymerization at a definite proportion or more. For example, a material containing 60% by mass or more, preferably 70% by mass or more, and more preferably 80% by mass or more of a browning reaction product with a molecular weight of 100,000 or more is preferably used. Also, a material containing 50% by mass or more, preferably 60% by mass or more, and more preferably 70% by mass or more of a browning reaction product with a molecular weight of 1,000,000 or more is preferably used. Alternatively, a browning reaction product with a weight-average molecular weight between 4,000,000 and 12,000,000, preferably between 5,000,000 and 10,000,000, and more preferably between 7,000,000 and 8,000,000 is preferably used.

The above-obtained browning reaction product may be used intact or subjected to elimination of low-molecular-weight components, desalting, and the like using a conventional purification means such as ultrafiltration and an ion exchange resin. Also, a browning reaction product may be precipitated by general means employed for selective precipitation of proteins, such as isoelectric point precipitation, salting-out, and organic solvent precipitation. A browning reaction product is preferably dried by a conventional means such as freeze-drying or spray drying, so as to facilitate formulation thereof or the addition thereof to food products and the like. When a browning reaction is carried out in a neutral aqueous solution, a browning reaction product may be directly dried, but is preferably dried and powdered after elimination of unreacted sugar and desalting. When a browning reaction is carried out in an aqueous alkaline solution, the reaction solution may be neutralized with acid (inorganic acid and/or organic acid). Furthermore, after neutralization, the product may be directly dried or dried and powdered after elimination of low-molecular-weight components and desalting.

Proteins to be used for production of browning reaction products are not limited, as long as they can be orally ingested. Examples of plant proteins include bean-derived proteins such as albumin and globulin, wheat-derived proteins, such as glutenin and albumin, and corn-derived proteins such as zein. Examples of animal proteins include milk-derived proteins such as casein or a salt thereof, lactoglobulin, lactalbumin, lactoferrin, albumin, and immunoglobulin, egg-derived proteins such as ovalbumin, ovotransferrin, ovomucoid, ovomucin, and lysozyme, and fish- or meat-derived proteins such as myosin and actin. In the present invention, as proteins to be used for browning reaction products, preferably milk-derived and particularly cow milk-derived proteins, and more preferably casein or a salt thereof, or preferably bean-derived proteins, and more preferably albumin and globulin can be used. As a salt of casein, a salt thereof that is generally used in the fields of foods or drinks can be used. Examples of such a salt of casein include, but are not limited to, alkali metal salts such as a sodium salt and a potassium salt, and alkaline-earth metal salts such as a calcium salt and a magnesium salt. Preferably, a sodium salt can be used.

These various proteins may be purified by conventional methods and then used or such proteins that are commercially available can also be directly used. These proteins may be used independently or in combination of two or more types thereof. Alternatively, food materials containing these proteins such as protein mixtures containing milk-derived proteins (e.g., row cow milk, milk powder, powdered skim milk, whey, and condensed milk) may be used herein.

Sugars to be used for browning reaction products are not particularly limited, as long as they can form browning reaction products with proteins. Such sugars may be any of monosaccharides, disaccharides, oligosaccharides, and polysaccharides. Examples of such sugars include various reducing sugars such as D-glucose, D-fructose, D-mannose, D-galactose, D-xylose, L-arabinose, D-ribose, maltose, and lactose. For production of browning reaction products in the present invention, preferably disaccharides and more preferably lactose can be used. These sugars may be used independently or in combination of two or more types thereof. Alternatively, food materials containing these sugars such as mixtures of sugars containing lactose (e.g., raw cow milk, milk powder, powdered skim milk, whey, and condensed milk) may be used.

Of the above examples, a browning reaction product (lactose casein polymerized product) prepared by reaction of casein or a salt thereof as a protein and lactose as a sugar is particularly preferred in terms of productivity and production efficiency.

The mass ratio of a sugar to a protein in a browning reaction product may be arbitrarily determined. The mass ratio generally ranges from 1:100 to 10:1 and preferably ranges from 1:9 to 1:1.

In the present invention, a browning reaction product can also be obtained by direct heat treatment or heat treatment in an aqueous solution of a food product containing a sugar and a protein and preferably a food product containing a sugar and a protein in the above mass ratio, such as raw cow milk, powdered cow milk, powdered skim milk, whey, condensed milk, or the like and thus carrying out a browning reaction, instead of mixing a sugar and a protein that are separately obtained and then causing a browning reaction in an aqueous solution. In this case, when a food material is used, a food material in the form of an aqueous suspension or an aqueous solution can be directly subjected to a browning reaction. However, a food material in a dry form is preferably suspended or dissolved in an aqueous solution and then subjected to heat treatment.

For example, powdered skim milk contains various milk proteins mainly composed of casein, and sugars such as lactose. Powdered skim milk can be obtained by separating and removing fat from raw milk by centrifugation according to a conventional method and then powderizing the thus generated delipidized milk. Commercially available powdered skim milk may also be used. A browning reaction is generally carried out by suspending powdered skim milk in an aqueous solution and then heating the suspension.

The above browning reaction product is an ingredient from a food material. The composition of the present invention can be widely and generally provided not only as a pharmaceutical composition, but also as a food product. Hence, the form of the composition of the present invention is not particularly limited. For example, the composition can be prepared as a pharmaceutical composition or a food product (including a feedstuff).

Examples of prevention of a peptic ulcer in the present invention include suppression of and delaying of the onset of diseases. Not only prevention before the disease is developed, but also prevention against relapse of the disease after treatment are included. Examples of treatment of a peptic ulcer in the present invention include the cure of the symptoms, improvement of the symptoms, and suppression of the further development of the symptoms.

The composition of the present invention can be administered to various animals and preferably mammals. The term "mammals" refers to homeotherms. Examples thereof include primates such as humans and monkeys, rodents such as mice, rats, and rabbits, pet animals such as dogs and cats, and domestic animals such as cattle, horses, and pigs. The composition of the present invention is preferably administered to primates, and particularly preferably to humans. Particularly preferably, the composition of the present invention is administered to a human who is afflicted with a peptic ulcer not caused by *Helicobacter pylori* and a human who may be afflicted with such peptic ulcer. Alternatively, the composition of the present invention is also preferably administered to a human who has no *Helicobacter pylori* but is afflicted with a peptic ulcer or a human who has been subjected to *Helicobacter pylori* eradication.

The composition of the present invention is administered to an adult per day in an amount ranging from 0.01 g to 20 g, preferably 0.1 g to 10 g, and more preferably 1 g to 3 g in dry mass based on the browning reaction product of a sugar and a protein, which is an active ingredient. The dosage can be further increased since the active ingredient of the composition of the present invention is derived from a food material and thus has high safety. Preferably the dosage is adequately increased or decreased in accordance with the effects and other conditions. The dosage per day can be preferably administered or ingested at once or in several separated doses.

When the composition of the present invention is prepared in the form of a pharmaceutical composition, in general, the composition is prepared as a pharmaceutical preparation comprising a browning reaction product that is an active ingredient and a pharmaceutically acceptable carrier. In general, the term "pharmaceutically acceptable carrier" refers to an inactive, atoxic, and solid or liquid extending agent, diluent, encapsulating material, or the like that does not react with the active ingredient of the present invention. Examples thereof include water, ethanol, polyol (e.g., glycerol, propylene glycol, or liquid polyethylene glycol), an appropriate mixture of any thereof, and a solvent or dispersion medium such as a vegetable oil. Also, additives that are conventionally used in production of pharmaceutical compositions can be added, as necessary, such as a solvent, a dispersant, an emulsifier, a buffering agent, a stabilizer, an excipient, a binder, a disintegrant, and a lubricant.

The dosage form of the pharmaceutical composition is not particularly limited, and an arbitrary dosage form can be employed. Examples thereof include dosage forms for oral administration, such as solid formulations (e.g., tablets (sugar-coated tablets, film-coated tablets, and the like), pills, granules, dust formulations, fine granules, capsules, soft capsules, and powdered drugs), semisolid formulations (e.g., gels and pastes), liquid formulations such as syrups, drinkable preparations, solutions, suspension solutions, fluid diet, and emulsification solutions, as well as capsules (e.g., hard capsules and soft capsules) and dosage forms for parenteral administration, such as sublingual tablets, nasal sprays, injection preparations, and suppositories.

Examples of techniques for administering the pharmaceutical composition of the present invention include administration techniques that are generally employed for pharmaceutical administration, such as intravenous administration, intramuscular administration, and subcutaneous administration, in addition to oral administration. Also, an administration technique that involves absorption through a mucous membrane other than the gastrointestinal tract, such as rectal, sublingual, or intranasal administration, can be employed. In such a case, the pharmaceutical composition can be administered in the form of, for example, a suppository, sublingual tablet, or nasal spray.

The content of a browning reaction product in a pharmaceutical composition varies depending on the dosage form thereof. The content is generally 1% by mass or more, preferably 3% by mass or more, more preferably 5% by mass or more, and further preferably 20% by mass or more, on a dry basis. The content is also generally 90% by mass or less, preferably 80% by mass or less, and more preferably 70% by mass or less, on a dry basis. The dosage form is preferably suitable for managing the dose per day, so as to realize the above-described dose for an adult per day. Since a browning reaction product has high safety, so that the content thereof can further be increased.

When the composition of the present invention is prepared in the form of food products, the form thereof is not particularly limited. Examples of food products include beverages, health food products, foods for specified health uses, and functional food products. Specifically, the health food product, the food for specified health use, and the functional food product can be prepared in the form of various pharmaceutical preparations, such as tablets (e.g., sugar-coated tablets and film-coated tablets), tablet sweets, chewable tablets, pills, granules, dust formulations, subtle granules, powdered drugs, gels, pastes, capsules, soft capsules, syrups, drinkable preparations, solutions, suspension solutions, emulsification solutions, suppositories, and fluid diet products. Specific examples of food products further include, but are not limited to, tea beverages such as green tea, oolong tea, and black tea, beverages such as soft drinks, jelly drinks, sports drinks, milk drinks, carbonated drinks, fruit juice beverages, lactic acid bacteria beverages, fermented milk drinks, powdered beverages, cocoa drinks, and purified water, spreads such as butter, jam, toppings, and margarine, mayonnaise, shortenings, custard cream, dressings, breads, cooked rice, noodles, pasta, miso soup, tofu, milk, yogurt, soup or sauces, and sweets (e.g., biscuits or cookies, chocolate, candies, cakes, ice cream, chewing gum, and tablets).

The composition of the present invention in the form of a food product can be produced by adding, in addition to a browning reaction product of a sugar and a protein, ingredients that are conventionally used for food products, such as various fats and oils, crude drugs, amino acids, polyhydric alcohol, naturally occurring polymers, vitamins, minerals, dietary fibers, surfactants, purified water, excipients, stabilizers, pH modifiers, antioxidants, sweeteners, taste components, acidulants, colorants, and aroma chemicals. The composition of the present invention in the form of a food product can also be prepared by mixing a browning reaction product of a sugar and a protein with a general food product. Such food product in the form of a pharmaceutical preparation can be produced in a manner similar to that for the above pharmaceutical composition. For example, the food product can be produced by adding an appropriate carrier (e.g., starch, processed starch, lactose, glucose, or water) and then using conventional techniques.

Furthermore, examples of the composition of the present invention include not only food products for humans, but also feedstuffs for domestic animals and racehorses, and pet foods. Feedstuffs are almost the same as food products except that they are used for subjects other than humans. Hence, descriptions concerning food products in this Description can be similarly applied to feedstuffs.

Regarding the composition of the present invention in the form of a food product, the content of a browning reaction product of a sugar and a protein differs depending on the form of the food product and is, on a dry basis, generally 1% by mass or more, preferably 5% by mass or more, further preferably 10% by mass or more, and further more preferably 20% by mass or more and is generally 80% by mass or less, preferably 70% by mass or less, and further preferably 60% by mass or less. Since a browning reaction product of a sugar and a protein has high safety, the content thereof can be further increased. The dosage per day can be preferably ingested at once or in several separated doses. The dosage form is preferably suitable for managing the dose per day, so as to realize the above-described dose for an adult per day.

In the composition of the present invention, food materials or pharmaceuticals that are used as effective ingredients for peptic ulcers can be incorporated or used in combination in order to promote the effects. Examples of such food materials or pharmaceuticals include colocynth, ignatia, lycopodium, strychnos, veratrum album, ginger, sodium azulenesulfonate, sodium alginate, L-glutamine, pirenzepine hydrochloride, ranitidine hydrochloride, omeprazole, famotidine, amylase preparation, dry yeast, diastase, orange peel tincture, pancreatin, swertia herb, dried aluminum hydroxide gel, magnesium silicate, and magnesium hydroxide.

### Examples

Hereinafter, the present invention will be described in detail with reference to Examples, but the present invention is not limited thereto. Examples 1-3 describe examples of "browning reaction products of sugars and proteins" contained in the compositions of the present invention and the production methods therefor.

### (Example 1) Browning reaction of Casein and lactose

Lactose (170 g) and 1500 g of casein were placed in a reaction vessel. After 15 L of purified water was added thereto and then they were dissolved, the pH was adjusted to 9 using NaOH. A browning reaction was carried out at 90°C for 8 hours while stirring the solution. After completion of the reaction, the resultant was neutralized and then freeze-dried, thereby obtaining 1680 g of browned powder. The browning reaction product accounted for approximately 90% of the powder.

The weight-average molecular weight of the thus obtained browning reaction product was 7,400,000. Also, when the molecular weight distribution was measured, the following results were obtained.

| | |
|---|---|
| 10,000,000 or more | 36.0% |
| 10,000,000-1,000,000 | 42.2% |
| 1,000,000-100,000 | 10.1% |
| 100,000 or less | 11. 6% |

### (Example 2) Browning reaction of Casein sodium and lactose

Sodium caseinate (60 g), 40 g of lactose, and 42 g of sodium hydrogencarbonate were added to 1 L of distilled water and then dissolved by stirring. A browning reaction was carried out at a temperature of 90°C for 5 hours. Desalting was carried out by ultrafiltration (Biomax-50, Millipore), followed by freeze-drying, so that 131 g of brown powder was obtained.

### (Example 3) Browsing reaction of Lactoglobulin and glucose

Lactoglobulin (3 g) and 2 g of glucose were dissolved in 50 mL of a 0.1 N-NaOH solution, followed by heating at 40°C for 3 hours. The reaction solution was neutralized with 1N-HCl. Low-molecular-weight components in the reaction solution were removed using an ultrafiltration membrane (Amicon Ultra-15 centrifugal filter device, molecular weight cutoff: 100,000 Da) and then the resultant was freeze-dried, so that 2.5 g of a browning reaction product was obtained.

### (Test example 1) Effects on NSAID (aspirin) ulcer (single dose)

SD rats were grouped (10 SD rats per group) by random sampling, so that the average body weights and dispersions of groups were almost the same. Then a test was conducted. The rats were fasted for 18 hours or more from the day before the test. The browning reaction product of Example 1 dissolved at 120 mg/mL in water for injection (Otsuka Pharmaceutical Factory Co., Ltd.) was orally administered using an oral sonde, so that the dose of the browning reaction product was 1.2 g/kg body weight. Thirty (30) minutes later, an aspirin suspension (prepared by suspension with 0.5% methylcellulose at 30 mg/mL aspirin) was orally administered using an oral sonde at 5 mL/kg body weight. A group of rats to which water for injection (Otsuka Pharmaceutical Factory Co., Ltd.) was administered instead of the browning reaction product was designated as a control group.

At 5 hours after administration of the aspirin suspension, rats were euthanized and then their stomachs were excised. The thus excised stomachs were fixed with formalin and then incised along the greater curvature. Ulcers of the mucosal surfaces were measured. Regarding ulcer length, the length of major axis was measured from among the major axis/minor axis of each ulcer, so as to find the ulcer length of each rat. The results are shown as the average value for each group in Table 1.

**Table 1**

| Group | Dose of browning reaction product (g/kg) | Length of long axis of ulcer (mm) |
|---|---|---|
| Control | 0 | 108.95 |
| Example 1 | 1.2 | 91.07 |

### (Test example 2) Effects on NSAID (aspirin) ulcer (repeated doses)

SD rats were grouped (10 rats per group) by random sampling, so that the average body weights and dispersions of the groups were almost the same. Then a test was conducted. The rats were fasted for 18 hours or more from the day before the test. The browning reaction product of Example 1 was orally administered using oral sondes at 0.1 g/kg body weight (dissolved at 10 mg/mL in water for injection), 0.3 g/kg body weight (dissolved at 30 mg/mL in water for injection), 1 g/kg body weight (dissolved at 100 mg/mL in water for injection), and 1.2 g/kg body weight (dissolved at 120 mg/mL in water for injection), respectively, for 8 days once a day. Thirty (30) minutes after administration of the browning reaction product on day 8, an aspirin suspension (prepared by suspension with 0.5% methylcellulose at 30 mg/mL aspirin) was orally administered using an oral sonde at 5 mL/kg body weight. A group of rats to which water for injection (Otsuka Pharmaceutical Factory Co., Ltd.) was administered instead of the browning reaction product was designated as a control group.

At 5 hours after administration of the aspirin suspension, rats were euthanized and then their stomachs were excised. The thus excised stomachs were fixed with formalin and then incised along the greater curvature. Ulcers of the mucosal surfaces were measured. Regarding ulcer length, the length of major axis was measured from among the major axis/minor axis of each ulcer, so as to find the ulcer length of each rat. The results are shown as the average value for each group in Table 2.

**Table 2**

| Group | Dose of browning reaction product (g/kg) | Length of long axis of ulcer (mm) |
|---|---|---|
| Control | 0 | 121.11 |
| Example 1 | 0.1 | 112.22 |
| | 0.3 | 109.89 |
| | 1 | 94.64 |
| | 1.2 | 95.01 |

### (Test example 3) Effects on stress ulcer due to water-immersion restraint (single dose)

SD rats were grouped (10 SD rats per group) by random sampling, so that the average body weights and dispersions of groups were almost the same. Then a test was conducted. The rats were fasted for 18 hours or more from the day before the test. The browning reaction product of Example 1 dissolved at 120 mg/mL in water for injection (Otsuka Pharmaceutical Factory Co., Ltd.) was orally administered using an oral sonde, so that the dose of the browning reaction product was 1.2 g/kg body weight. Thirty (30) minutes later, rats were housed in restraint stress cages (4.5×4.5×18 cm; 10 successive cages) and then immersed in a water bath at 23°C±1 to the height of the xiphisternum. A group of rats to which water for injection (Otsuka Pharmaceutical Factory Co., Ltd.) was administered instead of the browning reaction product was designated as a control group.

At 7 hours after water-immersion restraint, rats were euthanized and then their stomachs were excised. The thus excised stomachs were fixed with formalin and then incised along the greater curvature. Ulcers of the mucosal surfaces were measured. Regarding ulcer length, the length of major axis was measured from among the major axis/minor axis of each ulcer, so as to find the ulcer length of each rat. The results are shown as the average value for each group in Table 3.

**Table 3**

| Group | Dose of browning reaction product (g/kg) | Length of long axis of ulcer (mm) |
|---|---|---|
| Control | 0 | 30.77 |
| Example 1 | 1.2 | 20.20 |

### (Test example 4) Effects on stress ulcer due to water-immersion restraint (repeated doses)

SD rats were grouped (10 rats per group) by random sampling, so that the average body weights and dispersions of the groups were almost the same. Then a test was conducted. The rats were fasted for 18 hours or more from the day before the test. The browning reaction product of Example 1 was orally administered using oral sondes at 0.1 g/kg body weight (dissolved at 10 mg/mL in water for injection), 0.3 g/kg body weight (30 mg/mL browning reaction product), 1 g/kg body weight (100 mg/mL browning reaction product), and 1.2 g/kg body weight (120 mg/mL browning reaction product), respectively, once a day for 8 days. Thirty (30) minutes after administration of the browning reaction product on day 8, rats were housed in restraint stress cages (4.5×4.5×18 cm, 10 successive cages) and then immersed in a water bath at 23°C±1 to the height of the xiphisternum. A group of rats to which water for injection (Otsuka Pharmaceutical Factory Co., Ltd.) was administered instead of the browning reaction product was designated as a control group.

At 7 hours after water-immersion restraint, rats were euthanized and then stomachs were excised. The thus excised stomachs were fixed with formalin and then incised along the greater curvature. Ulcers of the mucosal surfaces were measured. Regarding ulcer length, the length of major axis was measured from among the major axis/minor axis of each ulcer, so as to find the ulcer length of each rat. The results are shown as the average value for each group in Table 4.

**Table 4**

| Group | Dose of browning reaction product (g/kg) | Length of long axis of ulcer (mm) |
|---|---|---|
| Control | 0 | 32.45 |
| Example 1 | 0.1 | 32.23 |
| | 0.3 | 24.74 |
| | 1 | 26.43 |
| | 1.2 | 25.73 |

The results of Test examples 1-4 revealed that both single dose and repeated doses of the composition of the present invention comprising as an active ingredient the browning reaction product of the sugar and the protein exerted excellent effects of reducing ulcer areas of aspirin-induced ulcers that are NSAID ulcers and the same of water-immersion restraint stress ulcers that are stress ulcers.

### (Example 4) Tablet

The browning reaction product (200 g) of Example 1, 210 g of crystalline cellulose, 50 g of lactose, 20 g of gum Arabic, and 20 g of hydroxypropyl cellulose were mixed. Ethanol (130 mL) was added into a kneading machine and then the mixture was kneaded by a general method for 5 minutes. After completion of kneading, the resultant was filtered using a 16-mesh filter and then dried at 50°C in a dryer. After drying, the dried product was granulated to obtain granules. Thirty (30) g of citric acid and 10 g of sucrose fatty acid ester were added to the granules. The mixture was mixed for approximately 1 minute and then tablets (300 mg per tablet) were prepared using a general tableting machine.

### (Example 5) Tablet and capsule

The browning reaction product (250 g) of Example 1, 580 g of crystalline cellulose, 118 g of reduced malt sugar, and 12.5 g of pectin were mixed. Sucrose fatty acid ester (40 g) (Mitsubishi Chemical Corporation) was further added to the mixture for mixing, thereby preparing powder. The powder was tableted using a tableting machine, so that tablets (500 mg per tablet) were prepared. Moreover, the powder was charged into No. 2 hard capsules, so that capsules were prepared.

### (Example 6) Fermented milk drink

In 7 kg of warm water (40°C), 800 g of powdered skim milk, 100 g of butter, 25 g of guar gum, and 150 g of the browning reaction product of Example 1 were dissolved and then warm water (40°C) was further added so that the total weight was 10 kg. The solution was homogenized (homogenization pressure: 100 kg/cm²), subjected to 3 minutes of heat sterilization at 90°C, homogenized again (homogenization pressure: 150 kg/cm²), and then cooled to 40°C. One (1) % by mass of yogurt starter culture was added to the solution and then the solution was maintained at 40°C to result in lactic acid acidity of 1% while agitating the solution. When the lactic acid acidity reached 1%, the solution was homogenized (homogenization pressure: 200 kg/cm²), cooled to 10°C, and then packed in portions of 200 ml each, so that lactic acid bacteria beverages were prepared.

### (Example 7) Soy milk drink

Carrageenan (10 g), 50 g of gum Arabic, and 150 g of the browning reaction product of Example 1 were dissolved in 2.5 kg of warm water (40°C). Also, 300 g of rice oil, 200 g of table sugar, and 50 g of common salt were added to and dissolved in 6.4 kg of soy milk (solid content: 11%) obtained from soybeans. Subsequently, the two solutions were mixed and then warm water was added so that the total weight thereof was 10 kg. The solution was homogenized (homogenization pressure: 200 kg/cm²), subjected to 5 seconds of heat sterilization at 140°C, homogenized again (homogenization pressure: 350 kg/cm²), cooled to 10°C, and then packed in portions of 200 ml each, so that soy milk was prepared.

### (Example 8) Cocoa drink

Locust bean gum (0.5 g) and 50 g of cocoa (Morinaga Milk Industry Co., Ltd) were added to 50 g of the browning reaction product of Example 1 and then the mixture was agitated. Water (approximately 80°C) (700 ml) was added and then the solution was agitated for suspension. After suspension, 200 ml of milk was added, so that cocoa drinks were prepared.

### (Example 9) Jelly drink

Xanthan gum (10 g), 5 g of carrageenan, 20 g of Tamarind seed gum, 50 g of calcium lactate, 20 g of potassium chloride, and 4 g of trisodium citrate were added sequentially to and dissolved in 5 kg of hot water (90°C or higher) while agitating the solution. After confirmation of dissolution, 166 g of the browning reaction product of Example 1 was dissolved in the solution. Moreover, 100 g of concentrated banana juice and 30 g of citric acid were dissolved in 3 kg of warm water (40°C). The two solutions were mixed, 500 g of table sugar was dissolved therein, 15 g of banana flavor was added, and then warm water (40°C) was added so that the total weight was 10 kg. The solution was subjected to 30 seconds of heat sterilization at 120°C, packed in portions of 180 ml each, and then refrigerated, so that jelly drinks were prepared.

### (Example 10) Chewable tablet

The browning reaction product (210 g) of Example 1, 453 g of lactose, 200 g of locust bean gum, 130 g of cocoa, 6 g of glycerin, and 1 g of sucrose were mixed, so that powder was prepared. The powder was tableted using a tableting machine, so that chewable tablets (1200 mg per tablet) were prepared.

All publications, patents, and patent applications cited herein are incorporated herein by reference in their entirety.

## Claims

1. A composition for preventing and/or treating a peptic ulcer not caused by *Helicobacter pylori,* comprising as an active ingredient a browning reaction product of a sugar and a protein.

2. The composition according to claim 1, wherein the peptic ulcer is an NSAID ulcer or a stress ulcer.

3. The composition according to claim 1 or 2, wherein the weight-average molecular weight of the browning reaction product of a sugar and a protein ranges from 4,000,000 to 12,000,000.
